(19) Europäisches Patentamt
European Patent Office
Office européen des brevets

(11) EP 2 793 801 B1

(12) **EUROPEAN PATENT SPECIFICATION**

(45) Date of publication and mention of the grant of the patent:
**24.07.2019 Bulletin 2019/30**

(21) Application number: **12808385.4**

(22) Date of filing: **20.12.2012**

(51) Int Cl.:
***A61K 8/19*** (2006.01) ***A61K 8/31*** (2006.01)
***A61Q 5/08*** (2006.01) ***A61K 8/34*** (2006.01)
***A61K 8/365*** (2006.01) ***A61K 8/20*** (2006.01)

(86) International application number:
**PCT/EP2012/076440**

(87) International publication number:
**WO 2013/092888 (27.06.2013 Gazette 2013/26)**

(54) **LIGHTENING PROCESS USING A COMPOSITION RICH IN FATTY SUBSTANCES AND METAL CATALYSTS, AND DEVICE SUITABLE THEREFOR**

BLEICHVERFAHREN MIT EINER FETTSTOFFREICHEN ZUSAMMENSETZUNG ENTHALTEND METALLKATALYSATOREN, UND ENTSPRECHENDE MEHRKOMPONENTENVERPACKUNGSEINHEIT

PROCÉDÉ D'ÉCLAIRCISSEMENT METTANT EN OEUVRE UNE COMPOSITION RICHE EN SUBSTANCES GRASSES ET COMPRENANT DES CATALYSEURS MÉTALLIQUES, ET DISPOSITIF APPROPRIÉ À CET EFFET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2011 FR 1162018**
**14.03.2012 US 201261610597 P**

(43) Date of publication of application:
**29.10.2014 Bulletin 2014/44**

(73) Proprietor: **L'Oréal**
**75008 Paris (FR)**

(72) Inventors:
- **SAMAIN, Henri**
  **F-91570 Bievres (FR)**
- **HERCOUET, Leïla**
  **F-93360 Neuilly Plaisance (FR)**
- **GIAFFERI, Marie**
  **F-93250 Villemonble (FR)**
- **LAGRANGE, Alain**
  **F-77700 Coupvray (FR)**
- **MIGNON, Marie**
  **F-75018 Paris (FR)**

(74) Representative: **Casalonga**
**Casalonga & Partners**
**Bayerstraße 71/73**
**80335 München (DE)**

(56) References cited:
**EP-A2- 2 263 642**
**WO-A1-03/047542**
**DE-A1-102006 012 575**
**DE-A1-102006 017 837**
**FR-A1- 2 946 875**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a process for lightening keratin fibres, in particular human keratin fibres such as the hair, comprising the use of one or more metal catalysts chosen from manganese salts, vanadium salts, cerium salts, and mixtures thereof and a composition (I) comprising at least 10% by weight of one or more fatty substances, one or more basifying agents and one or more oxidizing agents.

The invention also relates to a multi-compartment device suitable for performing the lightening process.

Processes for lightening human keratin fibres consist in using an aqueous composition comprising at least one oxidizing agent, under alkaline pH conditions in the vast majority of cases. The role of this oxidizing agent is to degrade the melanin of the hair, which, depending on the nature of the oxidizing agent present, leads to more or less pronounced lightening of the fibres. Thus, for relatively weak lightening, the oxidizing agent is generally hydrogen peroxide. When more substantial lightening is desired, peroxygenated salts, for instance persulfates, are usually used in the presence of hydrogen peroxide.

Lightening of the hair with a composition comprising at least 25% by weight of one or more fatty substances, one or more basifying agents, one or more surfactants, one or more oxidizing agents and water is achieved according to FR 2 946 875 A1. However, it is sought to further improve the level of lightening imparted to keratin fibres, especially when all or some of the ammonia is replaced with one or more other standard basifying agents.

It is known practice to use metal catalysts in hair lightening processes in order to reduce the amount of oxidizing agents used.

In particular, it has been observed that the use of metal catalysts led to curbing of the lightening of the hair.

There is thus a real need to propose lightening processes that do not have the drawbacks of the existing processes, i.e. which are capable of imparting a satisfactory level of lightening by using one or more metal catalysts.

This aim is achieved by the present invention, one subject of which is especially a process for lightening keratin fibres, in particular human keratin fibres such as the hair, which uses one or more metal catalysts chosen from manganese salts, vanadium salts, cerium salts, and mixtures thereof, and a composition (I) comprising:

(a) at least 10% of one or more fatty substances relative to the total weight of composition (I),
(b) one or more basifying agents, and
(c) one or more oxidizing agents.

The lightening process according to the invention leads to a satisfactory level of lightening, which is especially better than that obtained in a medium free of fatty substance or in a medium comprising little fatty substance.

In particular, the use of the lightening process according to the invention prevents the metal catalysts from curbing the lightening of keratin fibres, in contrast with the known lightening processes involving the use of metal catalysts.

In the process of the invention, the metal catalyst(s) may constitute or form part of a composition (A) which is mixed with composition (I) before applying the mixture to keratin fibres or which is applied separately as a pre-treatment or post-treatment with or without intermediate rinsing.

The present invention also relates to a multi-compartment device comprising a first compartment containing a cosmetic composition (A) comprising one or more metal catalysts chosen from manganese salts, vanadium salts, cerium salts, and mixtures thereof, a second compartment containing a cosmetic composition (B) comprising one or more basifying agents, and a third compartment comprising an oxidizing composition (C) comprising one or more oxidizing agents, at least one fatty substance being present in at least one of the compositions (B) or (C) such that, after mixing together compositions (B) and (C), the fatty substance content is greater than or equal to 10% by weight relative to the total weight of the mixture of compositions (B) and (C).

According to one particular embodiment, the device comprises a fourth compartment comprising a composition (D) comprising one or more fatty substances, the said composition (D) being intended to be mixed with compositions (B) and (C), the fatty substance content being greater than or equal to 10% by weight relative to the total weight of the mixture of compositions (B), (C) and (D), composition (B) or (C) optionally containing one or more fatty substances.

Other characteristics and advantages of the invention will emerge more clearly on reading the description and the examples that follow.

In the text hereinbelow, and unless otherwise indicated, the limits of a range of values are included in that range.

The expression "at least one" is equivalent to the expression "one or more".

The human keratin fibres treated via the process according to the invention are preferably the hair.

In addition, the process according to the invention is preferably not a dyeing process. It is thus preferably performed in the absence of direct dye or of oxidation dye precursor (bases and couplers). If they are present, their total content preferably does not exceed 0.005% by weight relative to the weight of each of the compositions. Specifically, at such a content, only the composition would be dyed, i.e. no dyeing effect would be observed on the keratin fibres.

Preferably, the process is performed without oxidation base or coupler or direct dye.

The lightening process according to the present invention uses one or more metal catalysts chosen from manganese salts, vanadium salts, cerium salts and mixtures thereof.

For the purposes of the present invention, the term "metal salts" means salts derived especially from the action of an acid on a metal.

The metal salts may be inorganic or organic salts.

The inorganic metal salts may be chosen from halides, carbonates, sulfates and phosphates, especially hydrated or anhydrous halides.

The organic metal salts may be chosen from organic acid salts such as citrates, lactates, glycolates, gluconates, acetates, propionates, fumarates, oxalates and tartrates, especially gluconates.

Preferentially, the metal catalysts are chosen from organic acid salts of manganese, inorganic salts of vanadium, and organic salts of cerium.

More preferentially, the metal catalysts are chosen from manganese gluconate, vanadium chloride and cerium chloride, optionally in hydrate form, such as cerium chloride heptahydrate.

According to one particularly preferred embodiment, the metal catalyst is manganese gluconate.

The metal catalysts may be present in a content ranging from 0.001% to 10% by weight and preferably in a content ranging from 0.001% to 1% by weight relative to the total weight of the composition applied to the keratin fibres.

The metal catalyst(s) may constitute all or part of a composition A.

This composition A may be anhydrous or aqueous.

When composition A is aqueous, the metal catalyst(s) may be present in a content ranging from 0.001% to 10% by weight, preferably in a content ranging from 0.001% to 1% by weight and better still ranging from 0.01% to 0.5% by weight relative to the total weight of the composition applied to the keratin fibres containing them.

As indicated previously, the lightening process is performed in the presence of a cosmetic composition (I) comprising at least 10% by weight of one or more fatty substances relative to the total weight of composition (I).

The term "fatty substance" means an organic compound that is insoluble in water at ordinary room temperature (25°C) and at atmospheric pressure (760 mmHg) (solubility of less than 5%, preferably 1% and even more preferentially 0.1%). They have in their structure at least one hydrocarbon-based chain containing at least 6 carbon atoms or a sequence of at least two siloxane groups. In addition, the fatty substances are generally soluble in organic solvents under the same temperature and pressure conditions, for instance chloroform, ethanol, benzene, liquid petroleum jelly or decamethylcyclopentasiloxane.

These fatty substances are neither polyoxyethylenated nor polyglycerolated. They are different from fatty acids, since salified fatty acids constitute soaps that are generally soluble in aqueous media.

The fatty substances are especially chosen from hydrocarbons comprising at least 6 carbon atoms and in particular $C_6$-$C_{16}$ alkanes, oils of animal origin, oils of plant origin, glycerides or fluoro oils of synthetic origin, fatty alcohols, fatty acid and/or fatty alcohol esters, non-silicone waxes and silicones.

It is recalled that, for the purposes of the invention, the fatty alcohols, fatty esters and fatty acids more particularly contain one or more linear or branched, saturated or unsaturated hydrocarbon-based groups comprising 6 to 30 carbon atoms, which are optionally substituted, in particular with one or more (in particular 1 to 4) hydroxyl groups. If they are unsaturated, these compounds may comprise one to three conjugated or unconjugated carbon-carbon double bonds.

As regards the $C_6$-$C_{16}$ alkanes, they are linear, branched or possibly cyclic. Examples that may be mentioned include hexane, dodecane, isoparaffins such as isohexadecane and isodecane. The linear or branched hydrocarbons of more than 16 carbon atoms may be chosen from liquid paraffins, petroleum jelly, liquid petroleum jelly, polydecenes, and hydrogenated polyisobutene such as Parleam®.

Among the animal oils, mention may be made of perhydrosqualene.

Among the triglycerides of plant or synthetic origin, mention may be made of liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, sunflower oil, castor oil, avocado oil, jojoba oil, shea butter oil and caprylic/capric acid triglycerides, for instance those sold by the company Stéarineries Dubois or those sold under the names Miglyol® 810, 812 and 818 by the company Dynamit Nobel.

Among the fluoro oils, mention may also be made of perfluoromethylcyclopentane and perfluoro-1,3-dimethylcyclohexane, sold under the names Flutec® PC1 and Flutec® PC3 by the company BNFL Fluorochemicals; perfluoro-1,2-dimethylcyclobutane; perfluoroalkanes such as dodecafluoropentane and tetradecafluorohexane, sold under the names PF 5050® and PF 5060® by the company 3M, or bromoperfluorooctyl sold under the name Foralkyl® by the company Atochem; nonafluoromethoxybutane and nonafluoroethoxyisobutane; perfluoromorpholine derivatives such as 4-(trifluoromethyl)perfluoromorpholine sold under the name PF 5052® by the company 3M.

The fatty alcohols that may be used in the cosmetic composition (I) are saturated or unsaturated, and linear or branched, and comprise from 6 to 30 carbon atoms and more particularly from 8 to 30 carbon atoms. Examples that may be mentioned include cetyl alcohol, stearyl alcohol and the mixture thereof (cetylstearyl alcohol), octyldodecanol,

2-butyloctanol, 2-hexyldecanol, 2-undecylpentadecanol, oleyl alcohol and linoleyl alcohol.

The wax(es) that may be used in the cosmetic composition (I) are chosen especially from carnauba wax, candelilla wax, esparto grass wax, paraffin wax, ozokerite, plant waxes, for instance olive wax, rice wax, hydrogenated jojoba wax or the absolute waxes of flowers such as the essential wax of blackcurrant blossom sold by the company Bertin (France), animal waxes, for instance beeswaxes, or modified beeswaxes (cerabellina); other waxes or waxy starting materials that may be used according to the invention are especially marine waxes such as the product sold by the company Sophim under the reference M82, and polyethylene waxes or polyolefin waxes in general.

As regards the esters of a fatty acid and/or of a fatty alcohol, which are advantageously different from the triglycerides mentioned above, mention may be made especially of esters of saturated or unsaturated, linear or branched $C_1$-$C_{26}$ aliphatic mono- or polyacids and of saturated or unsaturated, linear or branched $C_1$-$C_{26}$ aliphatic mono- or polyalcohols, the total carbon number of the esters more particularly being greater than or equal to 10.

Among the monoesters, mention may be made of dihydroabietyl behenate; octyldodecyl behenate; isocetyl behenate; cetyl lactate; $C_{12}$-$C_{15}$ alkyl lactate; isostearyl lactate; lauryl lactate; linoleyl lactate; oleyl lactate; (iso)stearyl octanoate; isocetyl octanoate; octyl octanoate; cetyl octanoate; decyl oleate; isocetyl isostearate; isocetyl laurate; isocetyl stearate; isodecyl octanoate; isodecyl oleate; isononyl isononanoate; isostearyl palmitate; methylacetyl ricinoleate; myristyl stearate; octyl isononanoate; 2-ethylhexyl isononate; octyl palmitate; octyl pelargonate; octyl stearate; octyldodecyl erucate; oleyl erucate; ethyl and isopropyl palmitates, 2-ethylhexyl palmitate, 2-octyldecyl palmitate, alkyl myristates such as isopropyl, butyl, cetyl, 2-octyldodecyl, myristyl or stearyl myristate, hexyl stearate, butyl stearate, isobutyl stearate; dioctyl malate, hexyl laurate, 2-hexyldecyl laurate.

Still within the context of this alternative form, use may also be made of esters of $C_4$-$C_{22}$ di- or tricarboxylic acids and of $C_1$-$C_{22}$ alcohols and esters of mono-, di- or tricarboxylic acids and of di-, tri-, tetra- or pentahydroxy $C_2$-$C_{26}$ alcohols.

Mention may in particular be made of: diethyl sebacate; diisopropyl sebacate; diisopropyl adipate; di(n-propyl) adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates.

Among the esters mentioned above, use is preferably made of ethyl, isopropyl, myristyl, cetyl or stearyl palmitate, 2-ethylhexyl palmitate, 2-octyldecyl palmitate, alkyl myristates, such as isopropyl, butyl, cetyl or 2-octyldodecyl myristate, hexyl stearate, butyl stearate, isobutyl stearate, dioctyl malate, hexyl laurate, 2-hexyldecyl laurate, isononyl isononanoate or cetyl octanoate.

The composition can also comprise, as fatty ester, sugar esters and diesters of $C_6$-$C_{30}$ and preferably $C_{12}$-$C_{22}$ fatty acids. It is recalled that the term "sugar" means oxygen-bearing hydrocarbon-based compounds containing several alcohol functions, with or without aldehyde or ketone functions, and which comprise at least 4 carbon atoms. These sugars can be monosaccharides, oligosaccharides or polysaccharides.

Mention may be made, as suitable sugars, for example, of sucrose (or saccharose), glucose, galactose, ribose, fucose, maltose, fructose, mannose, arabinose, xylose, lactose and their derivatives, in particular alkyl derivatives, such as methyl derivatives, for example methylglucose.

The esters of sugars and of fatty acids can be chosen in particular from the group consisting of the esters or mixtures of esters of sugars described above and of saturated or unsaturated and linear or branched $C_6$-$C_{30}$ and preferably $C_{12}$-$C_{22}$ fatty acids. If they are unsaturated, these compounds can comprise from one to three conjugated or non-conjugated carbon-carbon double bonds.

The esters according to this alternative form can also be chosen from mono-, di-, tri- and tetraesters, polyesters and their mixtures.

These esters can, for example, be oleates, laurates, palmitates, myristates, behenates, cocoates, stearates, linoleates, linolenates, caprates, arachidonates or their mixtures, such as, in particular, oleate/palmitate, oleate/stearate or palmitate/stearate mixed esters.

More particularly, use is made of mono- and diesters and in particular mono- or di-oleate, -stearate, -behenate, -oleate/palmitate, -linoleate, -linolenate or -oleate/stearate of sucrose, glucose or methylglucose.

Mention may be made, by way of example, of the product sold under the name Glucate® DO by Amerchol, which is a methylglucose dioleate.

Mention may also be made, by way of examples of esters or mixtures of esters of sugar and of fatty acid, of:

- the products sold under the names F160, F140, F110, F90, F70 and SL40 by Crodesta, respectively denoting sucrose palmitate/stearates formed of 73% monoester and 27% di- and triester, of 61% monoester and 39% di-, tri- and tetraester, of 52% monoester and 48% di-, tri- and tetraester, of 45% monoester and 55% di-, tri- and

tetraester, and of 39% monoester and 61% di-, tri- and tetraester, and sucrose monolaurate;

- the products sold under the name Ryoto Sugar Esters, for example referenced B370 and corresponding to sucrose behenate formed of 20% monoester and 80% diester, triester and polyester;
- the sucrose monopalmitate/stearate-dipalmitate/stearate sold by Goldschmidt under the name Tegosoft® PSE.

The silicones that can be used in the cosmetic composition (I) of the present invention are volatile or non-volatile, cyclic, linear or branched silicones, which are unmodified or modified with organic groups, having a viscosity from $5\times10^{-6}$ to 2.5 $m^2/s$ at 25°C, and preferably $1\times10^{-5}$ to 1 $m^2/s$.

The silicones which can be used in accordance with the invention can be provided in the form of oils, waxes, resins or gums.

Preferably, the silicone is chosen from polydialkylsiloxanes, in particular polydimethylsiloxanes (PDMSs), and organomodified polysiloxanes comprising at least one functional group chosen from poly(oxyalkylene) groups, amino groups and alkoxy groups.

Organopolysiloxanes are defined in more detail in Walter Noll's "Chemistry and Technology of Silicones" (1968), Academic Press. They can be volatile or non-volatile.

When they are volatile, the silicones are more particularly chosen from those having a boiling point of between 60°C and 260°C, and more particularly still from:

(i) cyclic polydialkylsiloxanes comprising from 3 to 7 and preferably from 4 to 5 silicon atoms. They are, for example, octamethylcyclotetrasiloxane, sold in particular under the name Volatile Silicone® 7207 by Union Carbide or Silbione® 70045 V2 by Rhodia, decamethylcyclopentasiloxane, sold under the name Volatile Silicone® 7158 by Union Carbide and Silbione® 70045 V5 by Rhodia, and mixtures thereof.

Mention may also be made of cyclocopolymers of the dimethylsiloxane/methylalkylsiloxane type, such as Volatile Silicone® FZ 3109, sold by Union Carbide, having the formula:

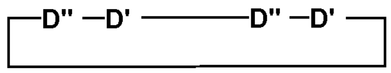

with D'' :

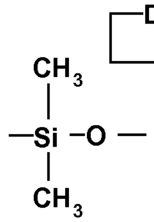

with D' :

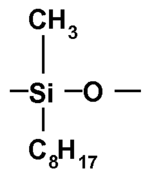

Mention may also be made of mixtures of cyclic polydialkylsiloxanes with organic compounds derived from silicon, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane;

(ii) linear volatile polydialkylsiloxanes containing 2 to 9 silicon atoms and having a viscosity of less than or equal to $5\times10^{-6}$ $m^2/s$ at 25°C. An example is decamethyltetrasiloxane sold in particular under the name SH 200 by the company Toray Silicone. Silicones coming within this category are also described in the paper published in Cosmetics and Toiletries, Vol. 91, Jan. 76, pp. 27-32, Todd & Byers, Volatile Silicone Fluids for Cosmetics.

Use is preferably made of non-volatile polydialkylsiloxanes, polydialkylsiloxane gums and resins, polyorganosiloxanes modified with the organofunctional groups above, and mixtures thereof.

These silicones are more particularly chosen from polydialkylsiloxanes, among which mention may be made mainly of polydimethylsiloxanes having trimethylsilyl end groups. The viscosity of the silicones is measured at 25°C according to Standard ASTM 445 Appendix C.

Mention may be made, among these polydialkylsiloxanes, without implied limitation, of the following commercial products:

- the Silbione® oils of the 47 and 70 047 series or the Mirasil® oils sold by Rhodia, such as, for example, the oil 70 047 V 500 000;
- the oils of the Mirasil® series sold by Rhodia;
- the oils of the 200 series from Dow Corning, such as DC200 having a viscosity of 60 000 $mm^2/s$;
- the Viscasil® oils from General Electric and certain oils of the SF series (SF 96, SF 18) from General Electric.

Mention may also be made of polydimethylsiloxanes having dimethylsilanol end groups known under the name

of dimethiconol (CTFA), such as the oils of the 48 series from Rhodia.

Mention may also be made, in this category of polydialkylsiloxanes, of the products sold under the names Abil Wax® 9800 and 9801 by Goldschmidt, which are polydi($C_1$-$C_{20}$)alkylsiloxanes.

The silicone gums which can be used in accordance with the invention are in particular polydialkylsiloxanes and preferably polydimethylsiloxanes having high number-average molecular weights of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane, tridecane or their mixtures.

Products which can be used more particularly in accordance with the invention are mixtures, such as:

- the mixtures formed from a polydimethylsiloxane hydroxylated at the chain end, or dimethiconol (CTFA), and from a cyclic polydimethylsiloxane, also known as cyclomethicone (CTFA), such as the product Q2 1401 sold by Dow Corning;
- the mixtures of a polydimethylsiloxane gum and of a cyclic silicone, such as the product SF 1214 Silicone Fluid from General Electric; this product is an SF 30 gum corresponding to a dimethicone, having a number-average molecular weight of 500 000, dissolved in the oil SF 1202 Silicone Fluid corresponding to decamethylcyclopentasiloxane;
- the mixtures of two PDMSs with different viscosities, and more particularly of a PDMS gum and a PDMS oil, such as the product SF 1236 from General Electric. The product SF 1236 is the mixture of a gum SE 30 defined above having a viscosity of 20 $m^2/s$ and of an oil SF 96 with a viscosity of $5\times10^{-6}$ $m^2/s$. This product preferably comprises 15% of gum SE 30 and 85% of an oil SF 96.

The organopolysiloxane resins which can be used in accordance with the invention are crosslinked siloxane systems including the following units:

$$R_2SiO_{2/2},\ R_3SiO_{1/2},\ RSiO_{3/2} \text{ and } SiO_{4/2}$$

in which R represents an alkyl having from 1 to 16 carbon atoms. Among these products, those that are particularly preferred are those in which R denotes a lower $C_1$-$C_4$ alkyl group, more particularly methyl.

Mention may be made, among these resins, of the product sold under the name Dow Corning 593 or those sold under the names Silicone Fluid SS 4230 and SS 4267 by General Electric, which are silicones of dimethyl/trimethylsiloxane structure.

Mention may also be made of the resins of the trimethylsiloxysilicate type, sold in particular under the names X22-4914, X21-5034 and X21-5037 by Shin-Etsu.

The organomodified silicones which can be used in accordance with the invention are silicones as defined above comprising, in their structure, one or more organofunctional groups attached via a hydrocarbon group.

In addition to the silicones described above, the organomodified silicones can be polydiarylsiloxanes, in particular polydiphenylsiloxanes, and polyalkylarylsiloxanes functionalized by the abovementioned organofunctional groups.

The polyalkylarylsiloxanes are chosen in particular from linear and/or branched polydimethyl/methylphenylsiloxanes and polydimethyl/diphenylsiloxanes with a viscosity ranging from $1\times10^{-5}$ to $5\times10^{-2}$ $m^2/s$ at 25°C.

Among these polyalkylarylsiloxanes, examples that may be mentioned include the products sold under the following names:

- Silbione® oils of the 70 641 series from Rhodia;
- the oils of the Rhodorsil® 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Mention may be made, among the organomodified silicones, of polyorganosiloxanes comprising:

- polyethyleneoxy and/or polypropyleneoxy groups optionally comprising $C_6$-$C_{24}$ alkyl groups, such as the products named dimethicone copolyol sold by Dow Corning under the name DC 1248 or the oils Silwet® L 722, L 7500, L 77 and L 711 by Union Carbide, and the ($C_{12}$)alkyl methicone copolyol sold by Dow Corning under the name Q2 5200;
- substituted or unsubstituted amino groups, such as the products sold under the names GP 4 Silicone Fluid and GP 7100 by Genesee or the products sold under the names Q2 8220 and Dow Corning 929 or 939 by Dow Corning. The substituted amino groups are in particular $C_1$-$C_4$ aminoalkyl groups;
- alkoxylated groups, such as the product sold under the name Silicone Copolymer F-755 by SWS Silicones, and

Abil Wax® 2428, 2434 and 2440 by Goldschmidt.

Preferably, the fatty substances do not comprise any $C_2$-$C_3$ oxyalkylene units or any glycerol units.

Preferably, the fatty substances are not fatty acids and in particular are not salified fatty acids or soaps, which are water-soluble compounds.

The fatty substances are advantageously chosen from $C_6$-$C_{16}$ hydrocarbons or hydrocarbons containing more than 16 carbon atoms and in particular alkanes, oils of plant origin, fatty alcohols, fatty acid and/or fatty alcohol esters, and silicones, or mixtures thereof.

Preferably, the fatty substance is an oil (a compound that is liquid at a temperature of 25°C and at atmospheric pressure).

Preferably, the fatty substance is chosen from liquid petrolatum, $C_6$-$C_{16}$ alkanes, polydecenes, liquid fatty acid and/or fatty alcohol esters, liquid fatty alcohols or their mixtures. Better still, the fatty substance is chosen from liquid petrolatum, $C_6$-$C_{16}$ alkanes or polydecenes.

The fatty substances are present in a content of greater than or equal to 10% by weight relative to the total weight of the cosmetic composition (I).

The cosmetic composition (I) has a fatty substance content preferably ranging from 10% to 70% by weight, even more particularly ranging from 20% to 70% by weight and better still from 25% to 70% by weight relative to the weight of composition (I).

The cosmetic composition (I) and/or composition (A) may also comprise one or more surfactants.

More particularly, the surfactant(s) are chosen from nonionic surfactants and anionic surfactants.

The term "anionic surfactant" means a surfactant comprising, as ionic or ionizable groups, only anionic groups. These anionic groups are preferably chosen from $CO_2H$, $CO_2^-$, $SO_3H$, $SO_3^-$, $OSO_3H$, $OSO_3^-$, $H_2PO_3$, $HPO_3^-$, $PO_3^{2-}$, $H_2PO_2$, $HPO_2$, $HPO_2^-$, $PO^{2-}$, POH and $PO^-$ groups.

As examples of anionic surfactants that may be used in the composition according to the invention, mention may be made of alkyl sulfates, alkyl ether sulfates, alkylamido ether sulfates, alkylaryl polyether sulfates, monoglyceride sulfates, alkyl sulfonates, alkylamide sulfonates, alkylaryl sulfonates, alpha-olefin sulfonates, paraffin sulfonates, alkyl sulfosuccinates, alkyl ether sulfosuccinates, alkylamide sulfosuccinates, alkyl sulfoacetates, acyl sarcosinates, acyl glutamates, alkyl sulfosuccinamates, acyl isethionates and N-acyltaurates, salts of alkyl monoesters of polyglycoside-polycarboxylic acids, acyl lactylates, D-galactoside-uronic acid salts, alkyl ether carboxylic acid salts, alkylaryl ether carboxylic acid salts, alkylamido ether carboxylic acid salts; and the corresponding non-salified forms of all these compounds; the alkyl and acyl groups of all these compounds comprising from 6 to 24 carbon atoms and the aryl group denoting a phenyl group.

These compounds can be oxyethylenated and then preferably comprise from 1 to 50 ethylene oxide units.

The salts of $C_{6-24}$ alkyl monoesters and polyglycoside-polycarboxylic acids may be selected from $C_{6-24}$ alkyl polyglycoside-citrates, $C_{6-24}$ alkyl polyglycoside-tartrates and $C_{6-24}$ alkyl polyglycoside-sulfo succinates.

When the anionic surfactant(s) are in salt form, they may be chosen from alkali metal salts, such as the sodium or potassium salt and preferably the sodium salt, ammonium salts, amine salts and in particular amino alcohol salts, or alkaline-earth metal salts, such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

Among the anionic surfactants, it is preferred, according to the invention, to use alkyl sulfate salts and alkyl ether sulfate salts, and alkyl ether carboxylic acids and salts thereof, and mixtures thereof.

The nonionic surfactants are chosen more particularly from mono- or polyoxyalkylenated or mono- or polyglycerolated nonionic surfactants. The oxyalkylene units are more particularly oxyethylene or oxypropylene units, or their combination, preferably oxyethylene units.

Examples of nonionic surfactants that can be used in the composition used according to the invention are described, for example, in the Handbook of Surfactants by M.R. Porter, published by Blackie & Son (Glasgow and London), 1991, pp. 116-178. They are especially chosen from alcohols, $\alpha$-diols and ($C_1$-$C_{20}$)alkylphenols, these compounds being polyethoxylated, polypropoxylated and/or polyglycerolated, and containing at least one fatty chain comprising, for example, from 8 to 40 carbon atoms, it being possible for the number of ethylene oxide and/or propylene oxide groups to especially range from 2 to 200, and for the number of glycerol groups to especially range from 2 to 30.

Mention may also be made of copolymers of ethylene oxide and propylene oxide, optionally oxyethylenated sorbitan fatty acid esters, sucrose fatty acid esters, polyoxyalkylenated fatty acid esters, optionally oxyalkylenated alkyl polyglycosides, alkyl glucoside esters, derivatives of N-alkyl glucamine and of N-acyl methylglucamine, aldobionamides, oxyethylenated and/or oxypropylenated silicones and amine oxides.

Examples of oxyalkylenated nonionic surfactants that may be mentioned include:

- oxyalkylenated ($C_8$-$C_{24}$)alkylphenols,
- saturated or unsaturated, linear or branched, oxyalkylenated $C_8$-$C_{30}$ alcohols,
- saturated or unsaturated, linear or branched, oxyalkylenated $C_8$-$C_{30}$ amides,
- esters of saturated or unsaturated, linear or branched, $C_8$-$C_{30}$ acids and of polyethylene glycols,
- polyoxyethylenated esters of saturated or unsaturated, linear or branched, $C_8$-$C_{30}$ acids and of sorbitol,
- oxyethylenated and saturated or unsaturated vegetable oils,
- condensates of ethylene oxide and/or of propylene oxide, inter alia,
- or mixtures thereof.

Preferably, the surfactants contain a number of moles of ethylene oxide and/or of propylene oxide of between 1 and 100 and preferably between 2 and 50. Advantageously, the nonionic surfactants do not comprise oxypropylene units.

As examples of monoglycerolated or polyglycerolated nonionic surfactants, monoglycerolated or polyglycerolated $C_8$-$C_{40}$ alcohols are preferably used.

In particular, the mono- or polyglycerolated $C_8$-$C_{40}$ alcohols correspond to the following formula:

$$RO\text{-}[CH_2\text{-}CH(CH_2OH)\text{-}O]_m\text{-}H$$

in which R represents a linear or branched $C_8$-$C_{40}$ and preferably $C_8$-$C_{30}$ alkyl or alkenyl radical and m represents a number ranging from 1 to 30 and preferably from 1 to 10.

Mention may be made, as examples of compounds which are suitable in the context of the invention, of lauryl alcohol comprising 4 mol of glycerol (INCI name: Polyglyceryl-4 Lauryl Ether), lauryl alcohol comprising 1.5 mol of glycerol, oleyl alcohol comprising 4 mol of glycerol (INCI name: Polyglyceryl-4 Oleyl Ether), oleyl alcohol comprising 2 mol of glycerol (INCI name: Polyglyceryl-2 Oleyl Ether), cetearyl alcohol comprising 2 mol of glycerol, cetearyl alcohol comprising 6 mol of glycerol, oleocetyl alcohol comprising 6 mol of glycerol, and octadecanol comprising 6 mol of glycerol.

The alcohol can represent a mixture of alcohols in the same way that the value of m represents a statistical value, which means that, in a commercial product, several types of polyglycerolated fatty alcohols can coexist in the form of a mixture.

Use is more preferably made, among the mono- or polyglycerolated alcohols, of the $C_8$/$C_{10}$ alcohol comprising 1 mol of glycerol, the $C_{10}$/$C_{12}$ alcohol comprising 1 mol of glycerol and the $C_{12}$ alcohol comprising 1.5 mol of glycerol.

In accordance with a preferred embodiment of the invention, the oxyalkylenated nonionic surfactants are chosen from oxyethylenated $C_8$-$C_{30}$ alcohols and polyoxyethylenated esters of saturated or unsaturated and linear or branched $C_8$-$C_{30}$ acids and of sorbitol.

Preferably, the cosmetic composition(I) comprises one or more nonionic surfactants.

The surfactant content of the compositions (I) and/or (A) more particularly represents from 0.1% to 50% by weight and preferably from 0.5% to 30% by weight relative to the weight of the composition under consideration.

Compositions (I) and/or (A) may also contain various adjuvants conventionally used in hair dye compositions, such as anionic, cationic, nonionic, amphoteric or zwitterionic polymers or mixtures thereof; mineral thickeners, and in particular fillers such as clays or talc; organic thickeners with, in particular, anionic, cationic, nonionic and amphoteric polymeric associative thickeners other than the polymers mentioned previously; antioxidants; penetrants; sequestrants; fragrances; dispersants; film-forming agents; ceramides; preserving agents; opacifiers.

The above adjuvants are generally present in an amount, for each of them, of between 0.01% and 20% by weight relative to the weight of the composition under consideration.

According to one advantageous variant of the invention, the composition (I) may also comprise one or more mineral or organic thickeners.

The mineral thickeners may be chosen from organophilic clays and mineral fillers.

The organophilic clay can be chosen from montmorillonite, bentonite, hectorite, attapulgite, sepiolite and their mixtures. The clay is preferably a bentonite or a hectorite.

These clays can be modified with a chemical compound chosen from quaternary ammoniums, tertiary amines, amine acetates, imidazolines, amine soaps, fatty sulfates, alkylarylsulfonates and amine oxides, and their mixtures.

Organophilic clays that may be mentioned include quaternium-18 bentonites, such as those sold under the

names Bentone 3, Bentone 38 and Bentone 38V by Rheox, Tixogel VP by United Catalyst and Claytone 34, Claytone 40 and Claytone XL by Southern Clay; stearalkonium bentonites, such as those sold under the names Bentone 27 by Rheox, Tixogel LG by United Catalyst and Claytone AF and Claytone APA by Southern Clay; quaternium-18/benzalkonium bentonites, such as those sold under the names Claytone HT and Claytone PS by Southern Clay; quaternium-18 hectorites, such as those sold under the names Bentone Gel DOA, Bentone Gel ECO5, Bentone Gel EUG, Bentone Gel IPP, Bentone Gel ISD, Bentone Gel SS71, Bentone Gel VS8 and Bentone Gel VS38 by Rheox, and Simagel M and Simagel SI 345 by Biophil.

Preferably, the mineral thickeners are chosen from inorganic fillers, in particular kaolinite.

The organic thickener(s) may be chosen from associative or non-associative thickening polymers, in particular associative thickening polymers.

According to the invention, the term "associative thickening polymer" means a thickening polymer comprising at least one $C_8$-$C_{30}$ fatty chain and at least one hydrophilic unit.

Preferably, the associative thickening polymers are chosen from polyurethane polyethers comprising in their chain both hydrophilic blocks usually of polyoxyethylenated nature and hydrophobic blocks, which may be aliphatic sequences alone and/or cycloaliphatic and/or aromatic sequences.

Such polyurethane polyethers are sold especially by the company Röhm & Haas under the names Aculyn 46® and Aculyn 44® [Aculyn 46® is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%); Aculyn 44® is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%)].

The non-associative thickening polymers may be chosen from celluloses.

Among the celluloses that are especially used are hydroxyethylcelluloses and hydroxypropylcelluloses. Mention may be made of the products sold under the names Klucel EF, Klucel H, Klucel LHF, Klucel MF and Klucel G by the company Aqualon, and Cellosize Polymer PCG-10 by the company Amerchol.

When they are present, the thickeners represent from 1% to 30% by weight relative to the weight of the composition.

Advantageously, the composition is in the form of a gel or a cream.

As indicated previously, composition (I) comprises one or more basifying agents.

The basifying agent(s) may be mineral or organic or hybrid.

For the purposes of the present invention, the term "mineral compound" means any compound bearing in its structure one or more elements from columns 1 to 13 of the Periodic Table of the Elements other than hydrogen.

The mineral basifying agent(s) are preferably chosen from aqueous ammonia, alkali metal carbonates or bicarbonates such as sodium or potassium carbonates and sodium or potassium bicarbonates, sodium hydroxide or potassium hydroxide, or mixtures thereof.

The organic alkaline agent(s) are preferably chosen from organic amines with a $pK_b$ at 25°C of less than 12, preferably less than 10 and even more advantageously less than 6. It should be noted that it is the $pK_b$ corresponding to the functional group of highest basicity. In addition, the organic amines do not comprise any alkyl or alkenyl fatty chains comprising more than ten carbon atoms.

According to the first variant of the invention, the organic amine comprises a primary, secondary or tertiary amine function and one or more linear or branched $C_1$-$C_8$ alkyl groups bearing one or more hydroxyl radicals.

Organic amines chosen from alkanolamines such as monoalkanolamines, dialkanolamines or trialkanolamines comprising one to three identical or different $C_1$-$C_4$ hydroxyalkyl radicals are in particular suitable for use.

Among compounds of this type, mention may be made of monoethanolamine, diethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol and tris(hydroxymethylamino)methane.

Also suitable are the organic amines of the following formula:

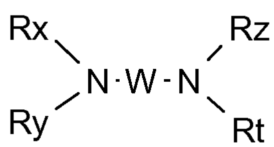

in which W is a $C_1$-$C_6$ alkylene residue optionally substituted with a hydroxyl group or a $C_1$-$C_6$ alkyl radical; Rx, Ry, Rz and Rt, which may be identical or different, represent a hydrogen atom or a $C_1$-$C_6$ alkyl, $C_1$-$C_6$ hydroxyalkyl or $C_1$-$C_6$ aminoalkyl radical.

Examples of such amines that may be mentioned include 1,3-diaminopropane, 1,3-diamino-2-propanol, spermine and spermidine.

According to a second variant of the invention, the organic amine is chosen from amino acids.

More particularly, the amino acids that can be used are of natural or synthetic origin, in their L, D or racemic form, and comprise at least one acid function chosen more particularly from carboxylic acid, sulfonic acid, phosphonic acid or phosphoric acid functions. The amino acids may be in neutral or ionic form.

As amino acids that can be used in the present invention, mention may be made especially of aspartic acid, glutamic acid, alanine, arginine, ornithine, citrulline, asparagine, carnitine, cysteine, glutamine, glycine, histidine, lysine, isoleucine, leucine, methionine, N-phenylalanine, proline, serine, taurine, threonine, tryptophan, tyrosine and valine.

Advantageously, the amino acids are basic amino acids comprising an additional amine function optionally included in a ring or in a ureido function.

Such basic amino acids are preferably chosen from those corresponding to formula (I) below:

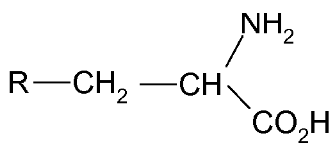

**(I)**

in which R denotes a group chosen from:

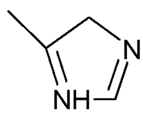

;

$-(CH_2)_3NH_2$; $-(CH_2)_2NH_2$; $-(CH_2)_2NHCONH_2$;

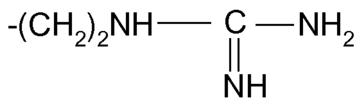

The compounds corresponding to formula (I) are histidine, lysine, arginine, ornithine and citrulline.

According to a third variant of the invention, the organic amine is chosen from organic amines of heterocyclic type. Besides histidine that has already been mentioned in the amino acids, mention may in particular be made of pyridine, piperidine, imidazole, triazole, tetrazole and benzimidazole.

According to a fourth variant of the invention, the organic amine is chosen from amino acid dipeptides.

As amino acid dipeptides that may be used in the present invention, mention may be made especially of carnosine, anserine and baleine.

According to a fifth variant of the invention, the organic amine is chosen from compounds comprising a guanidine function. Thus, the organic amine may be chosen from guanidine, arginine that has already been mentioned as an amino acid, creatine, creatinine, 1,1-dimethylguanidine, 1,1-diethylguanidine, glycocyamine, metformin, agmatine, N-amidinoalanine, 3-guanidinopropionic acid, 4-guanidinobutyric acid and 2-([amino(imino)methyl]amino)ethane-1-sulfonic acid.

Preferably, the organic amine(s) are chosen from alkanolamines, basic amino acids and compounds comprising a guanidine function.

In particular, the organic amine(s) are alkanolamines chosen from monoethanolamine, diethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol or tris(hydroxymethyl)aminomethane.

Preferably, the basifying agent(s) present in composition (I) of the invention are chosen from alkanolamines, amino acids in neutral or ionic form, in particular basic amino acids, and preferably corresponding to those of formula (I), and compounds comprising a guanidine function, and mixtures thereof.

Even more preferentially, the basifying agent(s) are chosen from alkanolamines such as monoethanolamine (MEA).

Advantageously, composition (I) according to the invention has a content of basifying agent(s) ranging from 0.01% to 30% by weight and preferably from 0.1% to 20% by weight relative to the weight of the said composition.

Compositions (I) and/or (A) may be anhydrous or aqueous.

For the purposes of the invention, the term "anhydrous cosmetic composition" means a cosmetic composition having a water content equal to 0 or less than 5% by weight, preferably less than 2% by weight and even more preferably less than 1% by weight relative to the weight of the said composition. It should be noted that the water in question is more particularly bound water, such as water of crystallization in salts, or traces of water absorbed by the raw materials used in the production of the compositions according to the invention.

The term "aqueous composition" is understood to mean a composition comprising more than 5% by weight of water, preferably more than 10% by weight of water and more advantageously still more than 20% by weight of water.

Preferably, the cosmetic composition (I) is an aqueous composition.

Even more preferentially, the water concentration may range from 10% to 90% and better still from 20% to 80% of the total weight of the composition.

Compositions (I) and/or (A) may also comprise one or more organic solvents.

Examples of organic solvents that may be mentioned include linear or branched $C_2$-$C_4$ alkanols, such as ethanol and isopropanol; glycerol; polyols and polyol ethers, for instance 2-butoxyethanol, propylene glycol, dipropylene glycol, propylene glycol monomethyl ether, diethylene glycol monomethyl ether and monoethyl ether, and also aromatic alcohols, for instance benzyl alcohol or phenoxyethanol, and mixtures thereof.

The organic solvent(s), if they are present, represent a content usually ranging from 1% to 40% by weight and preferably from 5% to 30% by weight relative to the weight of the cosmetic composition under consideration.

The pH of compositions (I) and/or (A), if they are aqueous, ranges from 2 to 13. For composition (I), it preferably ranges from 6.5 to 12 and better still from 8 to 12. The pH is adapted by using additional acidifying or basifying agents, such as those mentioned below.

Among the additional acidifying agents that may be mentioned, for example, are mineral or organic acids, for instance hydrochloric acid, orthophosphoric acid or sulfuric acid, carboxylic acids, for instance acetic acid, tartaric acid, citric acid and lactic acid, and sulfonic acids.

As regards the additional basifying agent optionally present in composition (A), it may be chosen from the non-salified organic amines described previously, or optionally, although not preferentially, aqueous ammonia.

According to a first particular embodiment, the compositions according to the invention preferably do not use aqueous ammonia, or a salt thereof, as basifying agent.

According to a second embodiment, if one of the compositions according to the invention does use aqueous ammonia, or a salt thereof, as basifying agent, its content should not exceed 0.03% by weight (expressed as $NH_3$) and preferably should not exceed 0.01% by weight, relative to the weight of the final composition resulting from the mixing together of the compositions.

Preferably, if the composition comprises aqueous ammonia, or a salt thereof, then the amount of basifying agent(s) other than the aqueous ammonia is greater than that of the aqueous ammonia (expressed as $NH_3$).

Finally, the process is performed with a composition (I) comprising one or more oxidizing agents.

More particularly, the oxidizing agent(s) are chosen from hydrogen peroxide, urea peroxide, alkali metal bromates or ferricyanides, peroxygenated salts, for instance alkali metal or alkaline-earth metal persulfates, perborates, peracids and precursors thereof, and percarbonates of alkali metals or alkaline-earth metals, and peracids and precursors thereof.

This oxidizing agent advantageously consists of hydrogen peroxide, in particular in aqueous solution (aqueous hydrogen peroxide solution), the concentration of which may vary, more particularly from 0.1% to 50% by weight, even more preferentially from 0.5% to 20% by weight and better still from 1% to 15% by weight relative to composition (I).

As indicated previously, composition (I) may result from the mixing of a composition (B) comprising one or more alkaline agents as defined previously and a composition (C) comprising one or more oxidizing agents as defined previously. Compositions (B) and (C) are preferably aqueous. They may especially be in the form of direct or inverse emulsions.

They may also result from the mixing of three compositions, the first two being compositions (B) and (C) above and the third composition being a composition (D) comprising at least one fatty substance as defined previously.

This composition (D) may be anhydrous or aqueous. It is preferably anhydrous.

Usually, the pH of the oxidizing composition (C), when it is aqueous, is less than 7.

In accordance with a first variant of the present invention, compositions (A) and (I) are mixed together, and the mixture made is then applied to wet or dry keratin fibres.

In a second variant of the invention, composition (A) and composition (I) which may result from the extemporaneous mixing of compositions (B), (C) and optionally (D) are applied successively to wet or dry keratin fibres, with or without intermediate rinsing.

Preferably, in this second variant, there is no intermediate rinsing.

Preferably, in this second variant, composition (A) is applied before composition (I).

The leave-on time of composition (A) on the keratin fibres may range from 5 to 15 minutes and is preferably 10 minutes.

In particular, composition (A) is applied to the keratin fibres and is left on for 10 minutes at room temperature.

Preferably, composition (A) is sprayed onto the keratin fibres.

In addition, composition (I), which may result from the mixing of compositions (B), (C) and optionally (D), may be left in place on the keratin fibres for a time generally from about 1 minute to 1 hour, preferably from 5 minutes to 40 minutes and preferably for 35 minutes.

The temperature during the process is conventionally between room temperature (between 15 and 25°C) and 80°C and preferably between room temperature and 60°C.

According to one preferred embodiment, composition (A) is applied to wet or dry keratin fibres and the fibres are then dried at a temperature ranging from room temperature to 60°C, in particular at 50°C, followed by the successive application, without intermediate rinsing, of composition (I) resulting from the extemporaneous mixing before application of compositions (B), (C) and optionally (D).

The drying step may last for 5 to 20 minutes and preferably for 5 to 15 minutes, and is especially 10 minutes.

After the treatment, the human keratin fibres are optionally rinsed with water, optionally washed with a shampoo and then rinsed with water, before being dried or left to dry.

Preferably, after the treatment, the keratin fibres are dried under a hood at a temperature ranging from 50 to 80°C.

According to one embodiment, the process for lightening keratin fibres comprises the use:

(a) of a composition (A) comprising one or more metal salts chosen from transition metal salts, in particular organic acid salts and inorganic salts of transition metals, and rare-earth metal salts, in particular inorganic salts, preferably manganese, vanadium and cerium salts,
(b) of a composition (B) comprising one or more alkaline agents chosen from alkanolamines,
(c) of a composition (C) comprising one or more oxidizing agents,
(d) of a composition (D) comprising one or more fatty substances chosen from liquid petroleum jelly, $C_6$-$C_{16}$ alkanes, polydecenes, liquid esters of fatty acids and/or of fatty alcohols, and liquid fatty alcohols, or mixtures thereof,

in which composition (A) is applied to the keratin fibres, followed by applying to the said fibres the composition resulting from the extemporaneous mixing of compositions (B), (C) and (D).

Finally, the invention relates to a multi-compartment device comprising a first compartment containing a cosmetic composition (A) comprising one or more metal catalysts as defined previously, a second compartment containing a cosmetic composition (B) comprising one or more basifying agents as defined previously, and a third compartment containing a composition (C) comprising one or more oxidizing agents as defined previously, at least one fatty substance as defined previously being present in at least one of the compositions (B) or (C) such that, after mixing together compositions (B) and (C), the fatty substance content is greater than or equal to 10% by weight relative to the total weight of the mixture of compositions (B) and (C).

According to one particular embodiment, the device comprises a fourth compartment comprising a composition (D) comprising one or more fatty substances, the said composition (D) being intended to be mixed with compositions (B) and (C), the fatty substance content being greater than or equal to 10% relative to the total weight of the mixture of compositions (B), (C) and (D), composition (B) or (C) optionally containing one or more fatty substances.

The device is suitable for implementing the lightening process.

The examples that follow serve to illustrate the invention without, however, being limiting in nature.

EXAMPLE 1

I. Compositions tested

1. Preparation of compositions based on metal salts

Compositions (A1), (A2) and (A3) based on metal salts are prepared (the amounts are expressed as weight percentages).

| Composition | A1 |
|---|---|
| Manganese gluconate | 0.1 |
| Demineralized water | qs 100 |
| pH | 6.52 |

| Composition | A2 |
|---|---|
| Cerium chloride heptahydrate | $8.2\times10^{-2}$ |
| pH | 5.46 |

| Composition | A3 |
|---|---|
| Vanadium chloride | $3.46\times10^{-2}$ |
| Demineralized water | qs 100 |
| pH | 2.76 |

2. Preparation of a lightening composition (I)

Compositions (B), (C) and (D) below are prepared (the amounts are expressed as weight percentages):

| Anhydrous composition | D |
|---|---|
| Oxyethylenated (2 OE) lauryl alcohol (Steareth-2) | 2 |
| (34/24/29/10 $C_8/C_{10}/C_{12}/C_{14}$)Alkyl polyglucoside (1,4) as an unprotected 53% aqueous solution (Plantacare 2000 UP - Cognis) | 1.96 |
| Demineralized water | 15 |
| Polymer of SMDI/polyethylene glycol bearing decyl end groups, as a 35% solution in water-glycol (Aculyn 44 - Röhm & Haas) | 0.5 |
| Kaolinite | 2.04 |
| Liquid petroleum jelly | qs 100 |

| Composition | B |
|---|---|
| Monoethanolamine | 15.1 |
| Hydroxyethyl cellulose | 1.5 |
| Sodium metabisulfite | 0.7 |
| Ascorbic acid | 0.25 |
| Diethylenetriaminepentaacetic acid | 1 |
| Propylene glycol | 6.2 |
| Ethanol | 8.25 |
| Hexylene glycol | 3 |
| Dipropylene glycol | 3 |
| Demineralized water | qs 100 |

| Oxidizing composition | C |
|---|---|
| Hydrogen peroxide | 6 |
| Liquid petrolatum | 20 |
| Cetearyl alcohol | 2.28 |

(continued)

| Oxidizing composition | C |
|---|---|
| Ceteareth-33 | 1.42 |
| Glycerol | 0.5 |
| Hydrogen peroxide stabilizers | 0.12 |
| Demineralized water | qs 100 |

At the time of use, 10 g of the anhydrous composition (D), 4 g of composition (B) and 15 g of composition (C) are mixed together to obtain a lightening composition (I). The pH of the lightening composition obtained is 9.7.

II. Procedure

Composition (A1), (A2) and (A3) based on metal salts is sprayed onto locks of Caucasian hair (tone depth = 4) which are placed vertically on a support. The "composition/lock" bath ratio is respectively 1/1 (g/g). The leave-on time is 10 minutes at ambient temperature.
The locks of hair are then dried under a hood at a temperature of 50°C for 10 minutes.
The lightening composition (I) is then applied to each of the locks by brush. The "composition/lock" bath ratio is respectively 1/1 (g/g). The locks of hair are again placed vertically on a support for 35 minutes at room temperature.
After this leave-on time, the locks of hair are washed with iNOA POST shampoo, rinsed and then dried under a hood at a temperature of 60°C.

III. Results

The lightening of the locks was evaluated in the CIE L* a* b* system, using a Minolta Spectrophotometer CM2600D colorimeter. In this L*, a*, b* system, L* represents the intensity of the colour, a* indicates the green/red colour axis and b* indicates the blue/yellow colour axis.

a. Calculation of the lightening value (DE*)

In the table below, the value of DE* is calculated from the values of L*a*b* according to equation (i) below:

$$DE^* = \sqrt{(L^* - L_o^*)^2 + (a^* - a_o^*)^2 + (b^* - b_o^*)^2} \quad (i)$$

In equation (i), L*, a* and b* represent the values measured on locks of hair after lightening and $L_0$*, $a_0$* and $b_0$* represent the values measured on locks of untreated hair.
The greater the value of DE*, the better the level of lightening.
The results are collated in the following tables:

Table - Lightening results (DE*)

| | L* | a* | b* | DE* |
|---|---|---|---|---|
| Lock of untreated hair | 21.45 | 2.08 | 1.98 | - |
| Lock of natural hair treated with composition (A1) and lightening composition (I) | 23.25 | 4.6 | 5.5 | 4.7 |
| Lock of hair treated with composition (A2) and the lightening composition (I) | 24.86 | 5.28 | 6.58 | 6.56 |
| Lock of hair treated with composition (A3) and the lightening composition (I) | 24.9 | 5.3 | 6.42 | 6.49 |

The process according to the invention leads to good lightening performance.

**Claims**

1. Process for lightening keratin fibres, in particular human keratin fibres such as the hair, comprising the use of one or more metal catalysts chosen from manganese salts, vanadium salts, cerium salts, and mixtures thereof, and of a composition (I) comprising:

   (a) at least 10% of one or more fatty substances relative to the total weight of composition (I),
   (b) one or more basifying agents,
   (c) one or more oxidizing agents.

2. Process according to Claim 1, **characterized in that** the metal salts are inorganic salts chosen from halides, carbonates, sulfates and phosphates, especially hydrated or anhydrous halides.

3. Process according to Claim 1, **characterized in that** the metal salts are organic acid salts chosen from citrates, lactates, glycolates, gluconates, acetates, propionates, fumarates, oxalates and tartrates, especially gluconates.

4. Process according to any one of the preceding claims, **characterized in that** the metal catalysts are chosen from organic acid salts of manganese, inorganic salts of vanadium, and inorganic salts of cerium.

5. Process according to any one of the preceding claims, **characterized in that** the fatty substances are chosen from oils that are liquid at room temperature and atmospheric pressure, preferably chosen from liquid petroleum jelly, polydecenes, liquid fatty alcohols, and liquid esters of fatty acids or of fatty alcohols, or mixtures thereof.

6. Process according to any one of the preceding claims, **characterized in that** the fatty substances are present in a content ranging from 10% to 70% by weight, even more particularly ranging from 20% to 70% by weight, better still from 25% to 70% by weight, even better still from 25% to 60% by weight and most particularly from 30% to 60% by weight relative to the total weight of composition (I).

7. Process according to any one of the preceding claims, **characterized in that** the composition (I) also comprises one or more surfactants, which are preferably nonionic.

8. Process according to any one of the preceding claims, **characterized in that** the basifying agents are chosen from organic amines with a $pK_b$ of less than 12, preferably less than 10 and more preferentially less than 6 at 25°C.

9. Process according to Claim 8, **characterized in that** the organic amine(s) are chosen from alkanolamines, basic amino acids and compounds comprising a guanidine function, and mixtures thereof, in particular alkanolamines.

10. Process according to any one of the preceding claims, **characterized in that** the oxidizing agent is hydrogen peroxide.

11. Process according to any one of the preceding claims, **characterized in that** a composition (A) comprising the said metal catalyst(s) and composition (I) are mixed together, and the mixture made is then applied to wet or dry keratin fibres.

12. Process according to any one of Claims 1 to 10, **characterized in that** a composition (A) comprising the said metal catalyst(s) and composition (I) are successively applied to wet or dry keratin fibres, with or without intermediate rinsing, preferably without intermediate rinsing.

13. Multi-compartment device comprising a first compartment containing a cosmetic composition (A) comprising one or more metal catalysts chosen from chosen from manganese salts, vanadium salts, cerium salts, and mixtures thereof, a second compartment containing a cosmetic composition (B) comprising one or more basifying agents, and a third compartment containing a composition (C) comprising one or more oxidizing agents, at least one fatty substance as defined in any one of Claims 1, 5 and 6 being present in at least one of the compositions (B) or (C) such that, after mixing together the compositions (B) and (C), the fatty substance content is greater than or equal to 10% by weight relative to the total weight of the mixture of compositions (B) and (C), and optionally a fourth compartment comprising a composition (D) comprising one or more fatty substances, the said composition (D) being intended to be mixed with compositions (B) and (C), the fatty substance content being greater than or equal to 10% by weight relative to the total weight of the mixture of compositions (B), (C) and (D), composition (B) or (C) optionally containing one or more fatty substances.

## Patentansprüche

1. Verfahren zum Aufhellen von Keratinfasern, insbesondere menschlichen Keratinfasern, wie z. B. des Haars, umfassend die Verwendung eines oder mehrerer Metallkatalysatoren ausgewählt aus Mangansalzen, Vanadiumsalzen, Cersalzen und Gemischen davon, und einer Zusammensetzung (I) umfassend:

   (a) wenigstens 10 % an einem oder mehreren Fettstoffen bezogen auf das Gesamtgewicht der Zusammensetzung (I),
   (b) ein oder mehrere Basifizierungsmittel,
   (c) ein oder mehrere Oxidationsmittel.

2. Verfahren gemäß Anspruch 1, **dadurch gekennzeichnet, dass** die Metallsalze anorganische Salze sind, ausgewählt aus Halogeniden, Carbonaten, Sulfaten und Phosphaten, insbesondere hydratisierten oder wasserfreien Halogeniden.

3. Verfahren gemäß Anspruch 1, **dadurch gekennzeichnet, dass** die Metallsalze Salze organischer Säuren sind, ausgewählt aus Citraten, Lactaten, Glycolaten, Gluconaten, Acetaten, Propionaten, Fumaraten, Oxalaten und Tartraten, insbesondere Gluconaten.

4. Verfahren gemäß einem der vorstehenden Ansprüche, **dadurch gekennzeichnet, dass** die Metallkatalysatoren ausgewählt sind aus Salzen organischer Säuren von Mangan, anorganischen Salzen von Vanadium und anorganischen Salzen von Cer.

5. Verfahren gemäß einem der vorstehenden Ansprüche, **dadurch gekennzeichnet, dass** die Fettstoffe ausgewählt sind aus Ölen, die bei Raumtemperatur und atmosphärischem Druck flüssig sind, vorzugsweise ausgewählt aus flüssigem Petrolatum, Polydecenen, flüssigen Fettalkoholen und flüssigen Estern von Fettsäuren oder von Fettalkoholen, und Gemischen davon.

6. Verfahren gemäß einem der vorstehenden Ansprüche, **dadurch gekennzeichnet, dass** die Fettstoffe in einem Gehalt in dem Bereich von 10 Gew.-% bis 70 Gew.-%, insbesondere in dem Bereich von 20 Gew.-% bis 70 Gew.-%, noch besser von 25 Gew.-% bis 70 Gew.-%, sogar noch besser von 25 Gew.-% bis 60 Gew.-% und vor allem von 30 Gew.-% bis 60 Gew.-%, bezogen auf das Gesamtgewicht der Zusammensetzung (I), vorhanden sind.

7. Verfahren gemäß einem der vorstehenden Ansprüche, **dadurch gekennzeichnet, dass** die Zusammensetzung (I) auch ein oder mehrere grenzflächenaktive Mittel, die vorzugsweise nichtionisch sind, umfasst.

8. Verfahren gemäß einem der vorstehenden Ansprüche, **dadurch gekennzeichnet, dass** die Basifizierungsmittel ausgewählt sind aus organischen Aminen mit einem $pK_b$-Wert von kleiner als 12, vorzugsweise kleiner als 10 und bevorzugter kleiner als 6 bei 25 °C.

9. Verfahren gemäß Anspruch 8, **dadurch gekennzeichnet, dass** die organischen Amine ausgewählt sind aus Alkanolaminen, basischen Aminosäuren und Verbindungen, die eine Guanidinfunktion umfassen, und Gemischen davon, insbesondere Alkanolaminen.

10. Verfahren gemäß einem der vorstehenden Ansprüche, **dadurch gekennzeichnet, dass** das Oxidationsmittel Wasserstoffperoxid ist.

11. Verfahren gemäß einem der vorstehenden Ansprüche, **dadurch gekennzeichnet, dass** eine Zusammensetzung (A), die den/die Metallkatalysator(en) umfasst, und die Zusammensetzung (I) zusammengemischt werden und das erhaltene Gemisch auf feuchte oder trockene Keratinfasern aufgebracht wird.

12. Verfahren gemäß einem der Ansprüche 1 bis 10, **dadurch gekennzeichnet, dass** eine Zusammensetzung (A), die den/die Metallkatalysator(en) umfasst, und die Zusammensetzung (I) aufeinanderfolgend auf feuchte oder trockene Keratinfasern aufgebracht werden, mit oder ohne Zwischenspülen, vorzugsweise ohne Zwischenspülen.

13. Mehrkammervorrichtung, umfassend eine erste Kammer, die eine kosmetische Zusammensetzung (A) enthält, die einen oder mehrere Metallkatalysatoren ausgewählt aus Mangansalzen, Vanadiumsalzen, Cersalzen und Gemischen davon umfasst, eine zweite Kammer, die eine kosmetische Zusammensetzung (B) enthält, die ein oder

mehrere Basifizierungsmittel umfasst, und eine dritte Kammer, die eine Zusammensetzung (C) enthält, die ein oder mehrere Oxidationsmittel umfasst, wobei wenigstens ein Fettstoff gemäß einem der Ansprüche 1 und 5 und 6 in wenigstens einer der Zusammensetzungen (B) und (C) vorhanden ist, so dass nach dem Zusammenmischen der Zusammensetzungen (B) und (C) der Fettstoffgehalt größer als oder gleich 10 Gew.-% bezogen auf das Gesamtgewicht des Gemischs der Zusammensetzungen (B) und (C) ist, und gegebenenfalls eine vierte Kammer, die eine Zusammensetzung (D) enthält, die einen oder mehrere Fettstoffe umfasst, wobei die Zusammensetzung (D) zum Mischen mit den Zusammensetzungen (B) und (C) vorgesehen ist, wobei der Fettstoffgehalt größer als oder gleich 10 Gew.-% bezogen auf das Gesamtgewicht des Gemischs der Zusammensetzungen (B), (C) und (D) ist, wobei die Zusammensetzung (B) oder (C) gegebenenfalls einen oder mehrere Fettstoffe enthält.

## Revendications

**1.** Procédé d'éclaircissement de fibres kératiniques, en particulier de fibres kératiniques humaines telles que des cheveux, dans lequel on met en oeuvre un ou plusieurs catalyseurs à base de métal, choisis parmi les sels de manganèse, les sels de vanadium, les sels de cérium et leurs mélanges, et une composition (I) comprenant :

a) au moins 10% d'un ou de plusieurs corps gras, en poids par rapport au poids total de la composition (I),
b) un ou plusieurs agents alcalinisants,
c) et un ou plusieurs agents oxydants.

**2.** Procédé selon la revendication 1, **caractérisé en ce que** les sels de métal sont des sels inorganiques choisis parmi les halogénures, carbonates, sulfates et phosphates, notamment les halogénures hydratés ou non hydratés.

**3.** Procédé selon la revendication 1, **caractérisé en ce que** les sels de métal sont des sels d'acide organique choisis parmi les citrates, lactates, glycolates, gluconates, acétates, propionates, fumarates, oxalates et tartrates, notamment les gluconates.

**4.** Procédé selon l'une des revendications précédentes, **caractérisé en ce que** les catalyseurs à base de métal sont choisis parmi les sels d'acide organique de manganèse, les sels inorganiques de vanadium et les sels inorganiques de cérium.

**5.** Procédé selon l'une des revendications précédentes, **caractérisé en ce que** les corps gras sont choisis parmi les huiles liquides à température ambiante et sous pression atmosphérique, et de préférence, parmi l'huile de vaseline, les polydécènes, les alcools gras liquides, les esters liquides d'acide gras ou d'alcool gras, et leurs mélanges.

**6.** Procédé selon l'une des revendications précédentes, **caractérisé en ce que** les corps gras sont présents en une teneur valant de 10 à 70% en poids, plus particulièrement de 20 à 70% en poids, mieux de 25 à 70% en poids, encore mieux de 25 à 60% en poids, et tout particulièrement de 30 à 60% en poids, par rapport au poids total de la composition (I).

**7.** Procédé selon l'une des revendications précédentes, **caractérisé en ce que** la composition (I) comprend en outre un ou plusieurs tensioactif(s), de préférence non-ionique(s).

**8.** Procédé selon l'une des revendications précédentes, **caractérisé en ce que** les agents alcalinisants sont choisis parmi les amines organiques présentant un $pK_b$ inférieur à 12, de préférence inférieur à 10 et mieux encore inférieur à 6, à 25 °C.

**9.** Procédé selon la revendication 8, **caractérisé en ce que** l'amine organique ou les amines organiques est ou sont choisie(s) parmi les alcanolamines, les acides aminés basiques et les composés comportant un groupe fonctionnel guanidine, ainsi que leurs mélanges, mais en particulier parmi les alcanolamines.

**10.** Procédé selon l'une des revendications précédentes, **caractérisé en ce que** l'agent oxydant est du peroxyde d'hydrogène.

**11.** Procédé selon l'une des revendications précédentes, **caractérisé en ce qu'**on mélange une composition (A) comprenant ledit ou lesdits catalyseur(s) à base de métal et la composition (I), puis on applique le mélange ainsi obtenu sur les fibres kératiniques, sèches ou mouillées.

**12.** Procédé selon l'une des revendications 1 à 10, **caractérisé en ce qu'**on applique successivement, sur les fibres kératiniques sèches ou mouillées, une composition (A) comprenant ledit ou lesdits catalyseur(s) à base de métal et la composition (I), avec ou sans rinçage intermédiaire, mais de préférence sans rinçage intermédiaire.

**13.** Dispositif à plusieurs compartiments, comportant un premier compartiment renfermant une composition cosmétique (A) comprenant un ou plusieurs catalyseur(s) à base de métal, choisi(s) parmi les sels de manganèse, les sels de vanadium et les sels de cérium, ainsi que leurs mélanges, un deuxième compartiment renfermant une composition cosmétique (B) comprenant un ou plusieurs agent(s) alcalinisants, et un troisième compartiment renfermant une composition (C) comprenant un ou plusieurs agent(s) oxydant(s), étant entendu qu'il y a au moins un corps gras, tel que défini dans l'une des revendications 1, 5 et 6, présent au moins dans l'une des compositions (B) et (C), de telle sorte qu'après mélangeage des compositions (B) et (C), la teneur en corps gras soit supérieure ou égale à 10 %, en poids rapporté au poids total du mélange des compositions (B) et (C), et en option, un quatrième compartiment renfermant une composition (D) comprenant un ou plusieurs corps gras, étant entendu que ladite composition (D) est à mélanger avec les compositions (B) et (C) et que la teneur en corps gras est alors supérieure ou égale à 10 %, en poids rapporté au poids total du mélange des compositions (B), (C) et (D), la composition (B) ou la composition (C) contenant, éventuellement, un ou plusieurs corps gras.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2946875 A1


### Non-patent literature cited in the description


- **WALTER NOLL'S.** Chemistry and Technology of Silicones. Academic Press, 1968
- *Cosmetics and Toiletries,* January 1976, vol. 91, 27-32
- **TODD ; BYERS.** *Volatile Silicone Fluids for Cosmetics*
- **M.R. PORTER.** Handbook of Surfactants. Blackie & Son, 1991, 116-178